# EUROPEAN PATENT APPLICATION

(11) **EP 4 477 168 A1**
(43) Date of publication of application: **18.12.2024**
(21) Application number: 22946527.3
(22) Date of filing: 11.10.2022
(51) Int. Cl.: A61B 18/12, A61B 18/14, A61B 10/02

(54) **RADIOFREQUENCY SCALPEL AND SYSTEM**

(30) Priority: 17.06.2022 CN 202210688318
(71) Applicant: Shanghai Cultiva Medical Device Co., Ltd., Shanghai 200120 (CN)
(72) Inventor: HE, Yihui, Shanghai 201210 (CN); SUN, Xiaoan, Shanghai 201210 (CN); CAO, Wenbin, Shanghai 201210 (CN)
(74) Representative: Argyma
(86) International application number: PCT/CN2022/124682
(87) International publication number: WO 2023/240855

(57) **Abstract**

The present invention provides a radiofrequency scalpel and system. The scalpel includes: a blade and a handle, where the blade includes: an outer blade, an inner blade, and a sheath. A distal end of the outer blade and a distal end of the inner blade are sequentially arranged from a distal end to a proximal end of the blade. The outer blade, the inner blade and the sheath are coaxial. The inner blade and the outer blade are configured to be capable of moving relatively in an axial direction or rotating relatively around a central axis. The inner blade includes an electrode. The electrode is connected to a radiofrequency generation apparatus. The electrode covers a cross-section of the distal end of the inner blade or covers a non-radial cross-sectional groove edge of an inner blade groove. The interior of the inner blade or an inner channel of the inner blade is configured to be connectable to a negative pressure apparatus. According to the present invention, cutting is realized by means of the inner blade and the outer blade which cooperate with each other, the scalpel cuts a large amount of tissue at a time, and the amount of bleeding is small during cutting.

## Description

### TECHNICAL FIELD

The present invention relates to the technical field of medical instruments, and in particular to a radiofrequency scalpel and system.

### BACKGROUND

Existing breast biopsy or other biopsy apparatuses use mechanical cutting, which mainly depends on a design structure and sharpness of a blade. The amount of tissue cut at a time is small, requiring multiple cutting and sampling, and a cutting process is accompanied by large bleeding. Therefore, in the prior art, operation time is long, and damage to a patient is great.

### SUMMARY

The present invention provides a radiofrequency scalpel and system, to solve the problems that the amount of tissue cut at a time is small and a cutting process is accompanied by large bleeding in the prior art.

According to a first aspect of the present invention, provided is a radiofrequency scalpel, including: a blade and a handle, where
the blade includes: an outer blade, an inner blade, and a sheath, and a distal end of the outer blade and a distal end of the inner blade are sequentially arranged from a distal end to a proximal end of the blade; the distal end of the blade is an end of the blade far away from the handle;
the outer blade, the inner blade, and the sheath are coaxial, and are separately connected to the handle;
a part of the inner blade penetrates through the sheath, and the distal end of the inner blade protrudes out of the sheath, the distal end of the inner blade being an end of the inner blade far away from the handle;
the inner blade and the outer blade are configured to be capable of moving relatively in an axial direction;
the inner blade includes: an electrode, the electrode being connected to a radiofrequency generation apparatus; the electrode covers a part or all of a cross section of the distal end of the inner blade; and
the interior of the inner blade or an inner channel of the inner blade is configured to be connectable to a negative pressure apparatus.

Preferably, the outer blade includes: a tip, an outer blade insulation layer, and a support rod;
the outer blade insulation layer is arranged at a proximal end of the tip, the proximal end of the tip being an end of the tip close to the handle;
the support rod is connected to a proximal end of the outer blade insulation layer, or penetrates through the outer blade insulation layer to be connected to the proximal end of the tip, the proximal end of the outer blade insulation layer being an end of the outer blade insulation layer far away from the tip;
theelectrode is sleeved outside the support rod;
that the inner blade and the outer blade are configured to be capable of moving relatively in an axial direction is specifically that the electrode and the support rod are configured to be capable of moving relatively in the axial direction; and
the inner blade includes an inner blade insulation layer, and the inner blade insulation layer covers a surface of the inner blade except the electrode.

Preferably, a sampling gap is reserved between an inner wall of the inner blade and an outer wall of the support rod, and the sampling gap is configured to be capable of serving as a part of an airflow channel of the negative pressure apparatus.

According to a second aspect of the present invention, provided is a radiofrequency scalpel, including: a blade and a handle, where
the blade includes: an outer blade, an inner blade, and a sheath; a distal end of the outer blade is located at a distal end of the blade, the distal end of the blade being an end of the blade far away from the handle, and the distal end of the outer blade being an end of the outer blade far away from the handle;
the outer blade, the inner blade, and the sheath are coaxial;
the inner blade and the sheath are separately connected to the handle, and a distal end of the sheath is fixedly connected to the outer blade; the distal end of the sheath is an end of the sheath far away from the handle;
the sheath is provided with a sheath groove at the distal end;
a distal end of the inner blade penetrates through the sheath, the inner blade and the sheath are configured to be capable of moving relatively in an axial direction, and during relative moving, the distal end of the inner blade is capable of entering the sheath groove;
the inner blade includes: an electrode, the electrode being connected to a radiofrequency generation apparatus; the electrode covers a part or all of a cross section of the distal end of the inner blade; and
the interior of the inner blade or an inner channel of the inner blade is configured to be connectable to a negative pressure apparatus.

Preferably, the outer blade includes: a tip and an outer blade insulation layer;
theouter blade insulation layer is arranged at a proximal end of the tip, the proximal end of the tip being an end of the tip close to the handle;
that the distal end of the sheath is fixedly connected to the outer blade is specifically that the distal end of the sheath is fixedly connected to the outer blade insulation layer; and
the inner blade includes an inner blade insulation layer, and the inner blade insulation layer covers a surface of the inner blade except the electrode.

Preferably, the radiofrequency scalpel further includes a sheath insulation layer, and the sheath insulation layer covers an edge of the sheath groove and an inner surface of the sheath.

Preferably, a sampling gap is reserved between an outer wall of the inner blade and an inner wall of the sheath, and the sampling gap is configured to be capable of serving as a part of an airflow channel of the negative pressure apparatus.

According to a third aspect of the present invention, provided is a radiofrequency scalpel, including: a blade and a handle, where
the blade includes: an outer blade, an inner blade, and a sheath; a distal end of the outer blade is located at a distal end of the blade, the distal end of the blade being an end of the blade far away from the handle, and the distal end of the outer blade being an end of the outer blade far away from the handle;
the outer blade, the inner blade, and the sheath are coaxial;
the inner blade and the sheath are separately connected to the handle, and a distal end of the sheath is fixedly connected to the outer blade; the distal end of the sheath is an end of the sheath far away from the handle;
a distal end of the inner blade penetrates through the sheath;
the inner blade is provided with an inner blade groove at the distal end, and the sheath is provided with a sheath groove at the distal end;
the inner blade includes: an electrode, the electrode is connected to a radiofrequency generation apparatus, the electrode covers a non-radial cross-sectional groove edge or a part of a non-radial cross-sectional groove edge of the inner blade groove, and the non-radial cross-sectional groove edge is a groove edge that is not located in a radial cross section of the inner blade;
the inner blade and the outer blade are configured to be capable of rotating relatively around a central axis of the blade, and during relative rotating, the electrode is capable of entering the sheath groove; and
theinterior of the inner blade or an inner channel of the inner blade is configured to be connectable to a negative pressure apparatus.

Preferably, the outer blade includes: a tip and an outer blade insulation layer;
the outer blade insulation layer is arranged at a proximal end of the tip, the proximal end of the tip being an end of the tip close to the handle;
that the distal end of the sheath is fixedly connected to the outer blade is specifically that the distal end of the sheath is fixedly connected to the outer blade insulation layer;
the inner blade includes: a support shaft and an inner blade insulation layer;
theinner blade groove is arranged at a distal end of the support shaft; and
theinner blade insulation layer covers a surface of the inner blade except the electrode.

Preferably, the radiofrequency scalpel further includes a sheath insulation layer, and the sheath insulation layer covers an edge of the sheath groove and an inner surface of the sheath.

Preferably, a sampling gap is reserved between an outer wall of the inner blade and an inner wall of the sheath, and the sampling gap is configured to be capable of serving as a part of an airflow channel of the negative pressure apparatus.

According to a fourth aspect of the present invention, provided is a radiofrequency surgical system, including a negative electrode plate, and the radiofrequency scalpel according to any one of the above items;
where the negative electrode plate and the radiofrequency scalpel are separately a part of a radiofrequency loop.

According to the radiofrequency scalpel and system provided by the present invention, through the combination of the outer blade and the inner blade, the inner blade and the outer blade are capable of moving relatively or rotating relatively. The inner blade is withdrawn, the radiofrequency generation apparatus is turned on, the electrode at the distal end of the inner blade starts to work, and tissue can be sucked in the vicinity of the electrode (between the inner blade and the outer blade). The inner blade is then fed to realize cutting with small cutting resistance and large amount of tissue cut at a time. Moreover, radiofrequency has a certain coagulation effect, and there is small bleeding during cutting.

In an alternative solution of the present invention, the sampling gap is reserved between the inner blade and the support rod or between the inner blade and the sheath, and the sampling gap is configured to be capable of serving as a part of the airflow channel of the negative pressure apparatus, to suck the cut tissue into a sample groove through negative pressure.

### BRIEF DESCRIPTION OF DRAWINGS

In order to explain the technical solutions in the embodiments of the present invention or in the prior art more clearly, the drawings used in the description of the embodiments or the prior art will be briefly introduced below. Clearly, the drawings in the following description are merely some embodiments of the present invention. For those of ordinary skill in the art, other drawings can be obtained based on these drawings without creative efforts.
FIG. 1 is a schematic diagram of a radiofrequency scalpel according to an embodiment of the present invention;
FIG. 2 is a schematic diagram and a partial enlarged diagram of blade withdrawing of a radiofrequency scalpel according to an embodiment of the present invention;
FIG. 3 is a schematic diagram of a radiofrequency scalpel according to another embodiment of the present invention;
FIG. 4 is a schematic diagram and a partial enlarged diagram of blade withdrawing of another radiofrequency scalpel according to another embodiment of the present invention;
FIG. 5 is a schematic diagram of a radiofrequency scalpel according to another embodiment of the present invention;
FIG. 6 is a schematic diagram and a partial enlarged diagram of blade withdrawing of another radiofrequency scalpel according to another embodiment of the present invention;
FIG. 7 is a schematic diagram of an inner blade according to an embodiment of the present invention;
FIG. 8 is a schematic diagram of an inner blade according to another embodiment of the present invention.
FIG. 9 is a schematic diagram of an inner blade according to another embodiment of the present invention.

### Description of reference signs:

1 - outer blade,
11 - tip,
12 - outer blade insulation layer,
13 - support rod,
2 - inner blade,
21 - electrode,
22 - inner blade insulation layer,
221 - first inner blade insulation layer,
222 - second inner blade insulation layer,
23 - inner blade groove,
24 - support shaft,
25 - support layer,
3 - sheath,
31 - sheath groove, and
32 - sheath insulation layer.

### DESCRIPTION OF EMBODIMENTS

The technical solutions in embodiments of the present invention will be clearly and fully described in combination with the drawings of the embodiments of the present invention; it is clear that the described embodiments are only a part of, and not all embodiments of, present invention. Based on the embodiments of the present invention, all other embodiments obtained by those of ordinary skill in the art without creative efforts should fall within the protection scope of the present invention.

In the description of the specification of the present invention, it should be understood that the term "upper portion", "lower portion", "upper end", "lower end", "upper surface", "lower surface" or the like indicates an orientation or positional relationship based on that shown in the drawings, which is merely for ease of description and simplicity of description, and is not intended to indicate or imply that the apparatus or element referred to must have a particular orientation, be constructed and operate in a particular orientation, and therefore cannot be construed as a limitation on the present invention.

In the description of the specification of the present invention, the terms "first" and "second" are used for descriptive purposes only, and cannot be construed as indicating or implying relative importance or implicitly indicating the number of technical features indicated. Therefore, a feature defined with "first" and "second" may explicitly or implicitly include one or more of the features.

In the description of the present invention, "a plurality of" means multiple, such as two, three, four or the like, unless specifically defined otherwise.

In the description of the specification of the present invention, unless otherwise expressly specified and limited, the terms "connect" and the like should be broadly understood, for example, they may be a fixed connection or a detachable connection or be integrated; or may be a mechanical connection or an electrical connection, or may communicate with each other; or may be a direct connection or an indirect connection through an intermediate medium, or may be a communication between the interior of two elements or the interaction of two elements. For those of ordinary skill in the art, the specific meanings of the above terms in the present invention can be understood according to specific situations.

The technical solutions of the present invention will be described in detail below by specific embodiments. The following several specific embodiments may be mutually combined, and same or similar concepts or processes may not be repeatedly described in some embodiments.

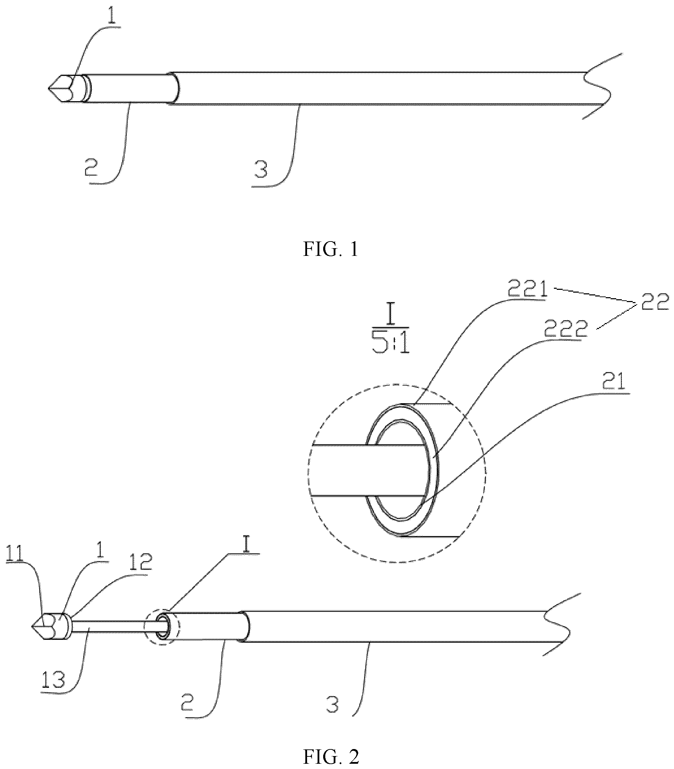

In an embodiment, provided is a radiofrequency scalpel, including: a blade and a handle (not shown in figures), where the blade includes: an outer blade 1, an inner blade 2, and a sheath 3. Refer to FIG. 1. A distal end of outer blade 1 and a distal end of the inner blade 2 are sequentially arranged from a distal end to a proximal end of the blade. The distal end of the blade is an end of the blade far away from the handle. The outer blade 1, the inner blade 2, and the sheath 3 are coaxial, and are separately connected to the handle. A part of the inner blade 2 penetrates through the sheath 3, and the distal end of the inner blade 2 protrudes out of the sheath 3. The distal end of the inner blade 2 is an end of the inner blade 2 far away from the handle. The inner blade 2 and the outer blade 1 are configured to be capable of moving relatively in an axial direction. The inner blade includes: an electrode 21, the electrode 21 being connected to a radiofrequency generation apparatus. The electrode 21 covers a part or all of a cross section of the distal end of the inner blade 2. Refer to FIG. 2. The interior of the inner blade or an inner channel of the inner blade is configured to be connectable to a negative pressure apparatus.

A work principle of the radiofrequency scalpel of the above embodiment is as follows: A negative electrode plate is placed at an appropriate position of a patient according to a surgical target area. The inner blade 2 moves in the axial direction relative to the outer blade 1, such that the electrode 22 of the inner blade 2 contacts the outer blade 1, that is, the inner blade is in a closed state. In such state, the radiofrequency scalpel is inserted into the surgical target area, and then the inner blade 2 operates in a direction far away from the outer blade 1, that is, the inner blade is withdrawn. During inner blade withdrawing or after the inner blade is withdrawn by a certain distance, the negative pressure apparatus is turned on. After the inner blade is withdrawn to a position, the inner blade 2 stops operating. In this case, tissue may be sucked in the vicinity of the electrode 22. The inner blade 2 operates in a direction toward the outer blade 1. During operation, the radiofrequency generation apparatus is turned on, and the electrode 22 transfers radiofrequency energy to the human tissue, that is, a cutting effect is achieved. The inner blade 1 operates, and the electrode 22 contacts the outer blade 1 again, that is, cutting is completed.

In an embodiment, the outer blade includes: a tip 11, an outer blade insulation layer 12, and a support rod 13. Refer to FIG. 2. The outer blade insulation layer 12 is arranged at a proximal end of the tip 11, the proximal end of the tip 11 being an end of the tip 11 close to the handle. The support rod 13 is connected to a proximal end of the outer blade insulation layer 12, or penetrates through the outer blade insulation layer 12 to be connected to the proximal end of the tip 11, the proximal end of the outer blade insulation layer 13 being an end of the outer blade insulation layer 13 far away from the tip 11.

The electrode 21 is sleeved outside the support rod 13. Furthermore, that the inner blade 2 and the outer blade 1 are configured to be capable of moving relatively in an axial direction is specifically that the electrode 21 and the support rod 13 are configured to be capable of moving relatively in the axial direction.

In addition, the inner blade includes: an inner blade insulation layer 22, and the inner blade insulation layer covers a surface of the inner blade except the electrode.

In an embodiment, the inner blade insulation layer 22 includes two layers: a first inner blade insulation layer 221 and a second inner blade insulation layer 222. The first inner blade insulation layer 221 covers an outer layer of the inner blade 2, and the second inner blade insulation layer 222 covers an inner layer of the inner blade 2. The outer layer of the inner blade is a layer of the inner blade far away from the support rod, and the inner layer of the inner blade is a layer of the inner blade close to the support rod.

In an embodiment, a sampling gap is reserved between an inner wall of the inner blade and an outer wall of the support rod. The sampling gap is configured to be capable of serving as a part of an airflow channel of the negative pressure apparatus, which provides a channel for the negative pressure apparatus to sample with negative pressure. With the negative pressure apparatus, a cut tissue sample can be sucked into a sample groove of the handle through the airflow channel.

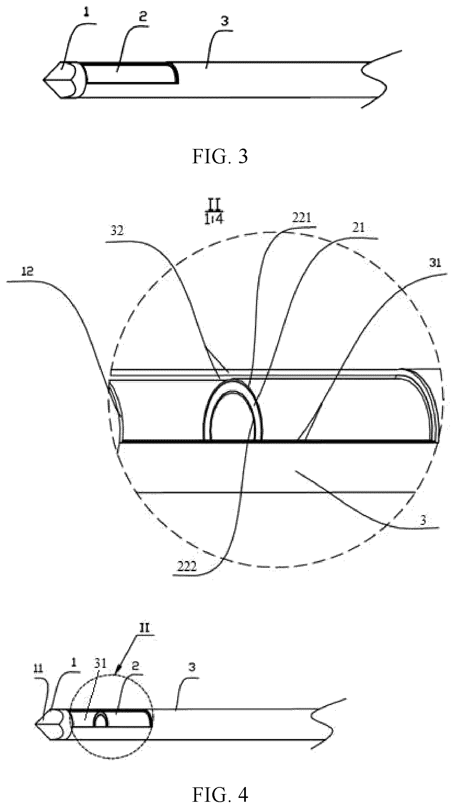

In another embodiment, provided is a radiofrequency scalpel, including: a blade and a handle (not shown in the figure), where the blade includes: an outer blade 1, an inner blade 2, and a sheath 3. Refer to FIG. 3. A distal end of the outer blade 1 is located at a distal end of the blade, the distal end of the blade being an end of the blade far away from the handle, and the distal end of the outer blade being an end of the outer blade far away from the handle. The outer blade 1, the inner blade 2, and the sheath 3 are coaxial. The inner blade 2 and the sheath 3 are separately connected to the handle. A distal end of the sheath 3 is fixedly connected to the outer blade 1. The distal end of the sheath 3 is an end of the sheath 3 far away from the handle. The sheath 3 is provided with a sheath groove 31 at the distal end. A distal end of the inner blade 2 penetrates through the sheath 3. The inner blade 2 and the sheath 3 are configured to be capable of moving relatively in an axial direction, and during relative moving, the distal end of the inner blade 2 is capable of entering the sheath groove 31. The inner blade 2 includes: an electrode 21, the electrode 21 being connected to a radiofrequency generation apparatus. The electrode 21 covers a part or all of a cross section of the distal end of the inner blade. Refer to FIG. 4. The interior of the inner blade or an inner channel of the inner blade is configured to be connectable to a negative pressure apparatus.

A work principle of the radiofrequency scalpel of the above embodiment is as follows: A negative electrode plate is placed at an appropriate position of a patient according to a surgical target area. The inner blade 2 moves in the axial direction relative to the outer blade 1, such that the electrode 22 of the inner blade 2 contacts the outer blade 1, that is, the inner blade is in a closed state. In such state, the radiofrequency scalpel is inserted into the surgical target area, and then the inner blade 2 operates in a direction far away from the outer blade 1, that is, the inner blade is withdrawn. During inner blade withdrawing or after the inner blade is withdrawn by a certain distance, the negative pressure apparatus is turned on. After the inner blade is withdrawn to a position, the inner blade 2 stops operating. In this case, tissue may be sucked in the vicinity of the electrode 22. The inner blade 2 operates in a direction toward the outer blade 1. During operation, the radiofrequency generation apparatus is turned on, and the electrode 22 transfers radiofrequency energy to the human tissue, that is, a cutting effect is achieved. The inner blade 1 operates, and the electrode 22 contacts the outer blade 1 again, that is, cutting is completed.

In an embodiment, the outer blade 1 includes: a tip 11 and an outer blade insulation layer 12. Refer to FIG. 4. The outer blade insulation layer 12 is arranged at a proximal end of the tip 11, the proximal end of the tip 11 being an end of the tip 11 close to the handle. Furthermore, that the distal end of the sheath 3 is fixedly connected to the outer blade is specifically that the distal end of the sheath 3 is fixedly connected to the outer blade insulation layer 12.

In addition, the inner blade 2 includes: an inner blade insulation layer, and the inner blade insulation layer covers a surface of the inner blade except the electrode.

In an embodiment, the inner blade insulation layer includes two layers: a first inner blade insulation layer 221 and a second inner blade insulation layer 222. The first inner blade insulation layer 221 covers an outer layer of the inner blade 2, and the second inner blade insulation layer 222 covers an inner layer of the inner blade 2. The outer layer of the inner blade is a layer of the inner blade close to the sheath 3, and the inner layer of the inner blade is a layer of the inner blade far away from the sheath 3.

In an embodiment, the radiofrequency scalpel further includes: a sheath insulation layer 32, and the sheath insulation layer 32 covers an edge of the sheath groove 31 and an inner surface of the sheath 3. Refer to FIG. 4.

In an embodiment, the sheath insulation layer 32 may be connected to the outer blade insulation layer 12, or the two may be of an integrated structure.

In an embodiment, a sampling gap is provided between an outer wall of the inner blade 2 and an inner wall of the sheath 3. The sampling gap is configured to be capable of serving as a part of an airflow channel of the negative pressure apparatus, which provides a channel for the negative pressure apparatus to sample with negative pressure. With the negative pressure apparatus, a cut tissue sample can be sucked into a sample groove of the handle through the airflow channel.

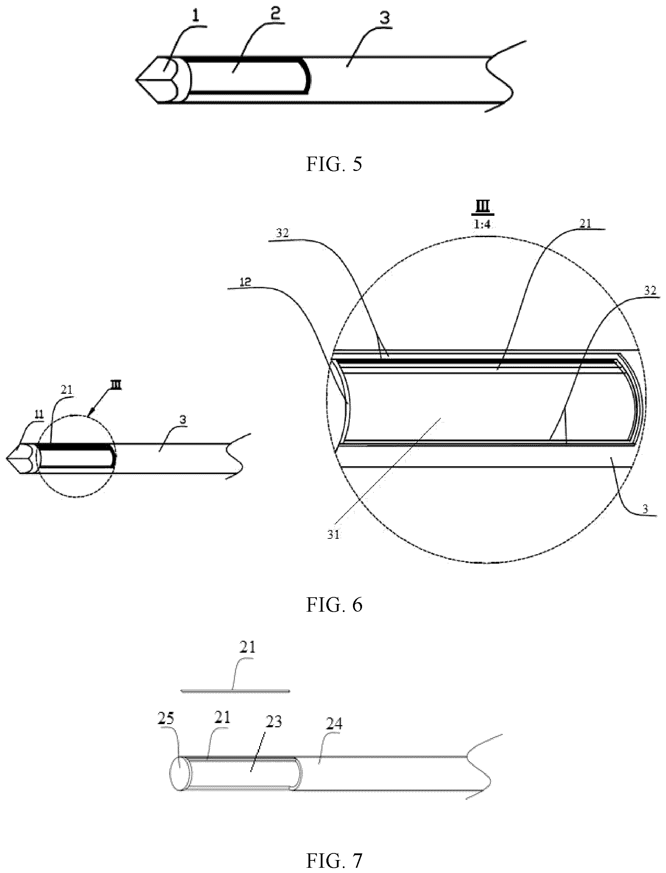

In another embodiment, provided is a radiofrequency scalpel, including: a blade and a handle (not shown in figures), where the blade includes: an outer blade 1, an inner blade 2, and a sheath 3. Refer to FIG. 5. A distal end of the outer blade 1 is located at a distal end of the blade, the distal end of the blade being an end of the blade far away from the handle, and the distal end of the outer blade 1 being an end of the outer blade far away from the handle. The outer blade 1, the inner blade 2, and the sheath 3 are coaxial. The inner blade 2 and the sheath 3 are separately connected to the handle. A distal end of the sheath 3 is fixedly connected to the outer blade 1. The distal end of the sheath 3 is an end of the sheath 3 far away from the handle. A distal end of the inner blade 2 penetrates through the sheath 3. The inner blade 2 is provided with an inner blade groove 23 at the distal end, and the sheath 3 is provided with a sheath groove 31 at the distal end. The inner blade 2 includes: an electrode 21, the electrode 21 being connected to a radiofrequency generation apparatus. The electrode 21 covers a non-radial cross-sectional groove edge or a part of a non-radial cross-sectional groove edge of the inner blade groove, and the non-radial cross-sectional groove edge is a groove edge that is not located in a radial cross section of the inner blade. Refer to FIG. 6. The inner blade 2 and the outer blade 1 are configured to be capable of rotating relatively around a central axis of the blade, and during relative rotating, the electrode 21 is capable of entering the sheath groove 31.

A work principle of the radiofrequency scalpel of the above embodiment is as follows: A negative electrode plate is placed at an appropriate position of a patient according to a surgical target area. The inner blade 2 rotates relative to the outer blade 1, such that the sheath groove 31 of the sheath 3 and the inner blade groove 23 of the inner blade 2 are staggered, that is, the sheath groove 31 is basically completely shielded by a surface of the inner blade 2, that is, the inner blade is in a closed state. In such state, the radiofrequency scalpel is inserted into the surgical target area, and then the inner blade 2 rotates again, that is, the inner blade is withdrawn. After the inner blade is withdrawn to a position where the electrode 21 is located at a side edge of the sheath groove and the inner blade groove 23 is opposite to the sheath groove 31, a negative pressure apparatus is turned on. In this case, tissue may be sucked into the inner blade groove 23, namely in the vicinity of the electrode 21. The inner blade 2 rotates, such that the electrode 21 passes through the sheath groove 31, and operates in a direction toward the other edge of the sheath groove 31. During operation, the radiofrequency generation apparatus is turned on, and the electrode 21 transfers radiofrequency energy to the human tissue, that is, a cutting effect is realized. The electrode 21 rotates to a position on the other edge of the sheath groove 31, namely a position where the sheath groove 31 is basically completely shielded by the surface of the inner blade, that is, cutting is completed.

In an embodiment, the outer blade 1 includes: a tip 11 and an outer blade insulation layer 12. Refer to FIG. 6. The outer blade insulation layer 12 is arranged at a proximal end of the tip 11, the proximal end of the tip 11 being an end of the tip 11 close to the handle. Furthermore, that the distal end of the sheath 3 is fixedly connected to the outer blade 1 is specifically that the distal end of the sheath 3 is fixedly connected to the outer blade insulation layer 12.

In addition, the inner blade 2 includes: a support shaft 24. Refer to FIG. 6 . The inner blade groove 23 is provided at a distal end of the support shaft 24.

In an embodiment, the radiofrequency scalpel further includes: a sheath insulation layer 32, and the sheath insulation layer 32 covers an edge of the sheath groove 31 and an inner surface of the sheath 3. Refer to FIG. 6.

In an embodiment, a support layer 25 is further arranged on one side of the inner blade groove close to the outer blade, for supporting the electrode 21. In this case, two ends of the electrode 21 are respectively fixed on the support shaft 24 and the support layer 25 on two sides of the inner blade groove. Refer to FIG. 7.

In an embodiment, the support shaft 24 and the support layer 25 are made of insulation material, or are wrapped with insulation layers, which are insulators.

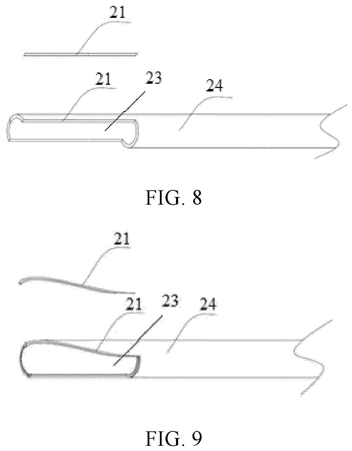

In a different embodiment, the support layer may not be included, and the electrode 21 may be directly attached to the non-radial cross-sectional groove edge of the inner blade groove. Refer to FIG. 8, and FIG. 9.

When the electrode 21 is directly attached to the non-radial cross-sectional groove edge of the inner blade groove, the inner blade may further include: an inner blade insulation layer, and the inner blade insulation layer covers a surface of the inner blade 2 except the electrode.

In an embodiment, the electrode 21 may be of a straight rod-shaped structure, referring to FIG. 7; the electrode may be composed of a straight rod and an elbow, referring to FIG. 8; or the electrode may be of an elbow-shaped structure, referring to FIG. 9, provided that a cutting action can be completed.

In addition, the shape of the inner blade groove 23 may also be designed differently according to actual needs. Refer to FIG. 7, FIG. 8, and FIG. 9.

In an embodiment, a sampling gap is reserved between an outer wall of the inner blade and an inner wall of the sheath. The sampling gap is configured to be capable of serving as a part of an airflow channel of the negative pressure apparatus, which provides a channel for the negative pressure apparatus to sample with negative pressure. With the negative pressure apparatus, a cut tissue sample can be sucked into a sample groove of the handle through the airflow channel.

In another embodiment, provided is a radiofrequency surgical system, including a negative electrode plate, and a radiofrequency scalpel according to any above embodiment. The negative electrode plate, human tissue, and the radiofrequency scalpel form a radiofrequency loop.

In the description of this specification, description of reference terms such as "an implementation", "an embodiment", "a specific implementation process", or "an example" means including specific features, structures, materials, or characteristics described in the embodiment or example in at least one embodiment or example of the present invention. In this specification, exemplary descriptions of the foregoing terms do not necessarily refer to the same embodiment or example. Furthermore, specific features, structures, materials, or characteristics described may be combined in any suitable manner in one or more embodiments or examples.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without making the essence of the corresponding technical solutions departing from the scope of the technical solutions of the embodiments of the present invention.

## Claims

1. A radiofrequency scalpel, comprising: a blade and a handle, wherein
the blade comprises: an outer blade, an inner blade, and a sheath, and a distal end of the outer blade and a distal end of the inner blade are sequentially arranged from a distal end to a proximal end of the blade; the distal end of the blade is an end of the blade far away from the handle;
the outer blade, the inner blade, and the sheath are coaxial, and are separately connected to the handle;
a part of the inner blade penetrates through the sheath, and the distal end of the inner blade protrudes out of the sheath, the distal end of the inner blade being an end of the inner blade far away from the handle;
the inner blade and the outer blade are configured to be capable of moving relatively in an axial direction;
the inner blade comprises: an electrode, the electrode being connected to a radiofrequency generation apparatus; the electrode covers a part or all of a cross section of the distal end of the inner blade; and
the interior of the inner blade or an inner channel of the inner blade is configured to be connectable to a negative pressure apparatus.

2. The radiofrequency scalpel according to claim 1, wherein the outer blade comprises: a tip, an outer blade insulation layer, and a support rod;
the outer blade insulation layer is arranged at a proximal end of the tip, the proximal end of the tip being an end of the tip close to the handle;
the support rod is connected to a proximal end of the outer blade insulation layer, or penetrates through the outer blade insulation layer to be connected to the proximal end of the tip, the proximal end of the outer blade insulation layer being an end of the outer blade insulation layer far away from the tip;
the electrode is sleeved outside the support rod;
that the inner blade and the outer blade are configured to be capable of moving relatively in an axial direction is specifically that the electrode and the support rod are configured to be capable of moving relatively in the axial direction; and
the inner blade comprises an inner blade insulation layer, and the inner blade insulation layer covers a surface of the inner blade except the electrode.

3. The radiofrequency scalpel according to claim 2, wherein a sampling gap is reserved between an inner wall of the inner blade and an outer wall of the support rod, and the sampling gap is configured to be capable of serving as a part of an airflow channel of the negative pressure apparatus.

4. A radiofrequency scalpel, comprising: a blade and a handle, wherein
the blade comprises: an outer blade, an inner blade, and a sheath; a distal end of the outer blade is located at a distal end of the blade, the distal end of the blade being an end of the blade far away from the handle, and the distal end of the outer blade being an end of the outer blade far away from the handle;
the outer blade, the inner blade, and the sheath are coaxial;
the inner blade and the sheath are separately connected to the handle, and a distal end of the sheath is fixedly connected to the outer blade; the distal end of the sheath is an end of the sheath far away from the handle;
the sheath is provided with a sheath groove at the distal end;
a distal end of the inner blade penetrates through the sheath, the inner blade and the sheath are configured to be capable of moving relatively in an axial direction, and during relative moving, the distal end of the inner blade is capable of entering the sheath groove;
the inner blade comprises: an electrode, the electrode being connected to a radiofrequency generation apparatus; the electrode covers a part or all of a cross section of the distal end of the inner blade; and
the interior of the inner blade or an inner channel of the inner blade is configured to be connectable to a negative pressure apparatus.

5. The radiofrequency scalpel according to claim 4, wherein the outer blade comprises: a tip and an outer blade insulation layer;
the outer blade insulation layer is arranged at a proximal end of the tip, the proximal end of the tip being an end of the tip close to the handle;
that the distal end of the sheath is fixedly connected to the outer blade is specifically that the distal end of the sheath is fixedly connected to the outer blade insulation layer; and
the inner blade comprises an inner blade insulation layer, and the inner blade insulation layer covers a surface of the inner blade except the electrode.

6. The radiofrequency scalpel according to claim 5, further comprises: a sheath insulation layer, the sheath insulation layer covering an edge of the sheath groove and an inner surface of the sheath.

7. The radiofrequency scalpel according to any one of claims 4-6, wherein a sampling gap is reserved between an outer wall of the inner blade and an inner wall of the sheath, and the sampling gap is configured to be capable of serving as a part of an airflow channel of the negative pressure apparatus.

8. A radiofrequency scalpel, comprising: a blade and a handle, wherein
the blade comprises: an outer blade, an inner blade, and a sheath; a distal end of the outer blade is located at a distal end of the blade, the distal end of the blade being an end of the blade far away from the handle, and the distal end of the outer blade being an end of the outer blade far away from the handle;
the outer blade, the inner blade, and the sheath are coaxial;
the inner blade and the sheath are separately connected to the handle, and a distal end of the sheath is fixedly connected to the outer blade; the distal end of the sheath is an end of the sheath far away from the handle;
a distal end of the inner blade penetrates through the sheath;
the inner blade is provided with an inner blade groove at the distal end, and the sheath is provided with a sheath groove at the distal end;
the inner blade comprises: an electrode, the electrode is connected to a radiofrequency generation apparatus, the electrode covers a non-radial cross-sectional groove edge or a part of a non-radial cross-sectional groove edge of the inner blade groove, and the non-radial cross-sectional groove edge is a groove edge that is not located in a radial cross section of the inner blade;
the inner blade and the outer blade are configured to be capable of rotating relatively around a central axis of the blade, and during relative rotating, the electrode is capable of entering the sheath groove; and
the interior of the inner blade or an inner channel of the inner blade is configured to be connectable to a negative pressure apparatus.

9. The radiofrequency scalpel according to claim 8, wherein the outer blade comprises: a tip and an outer blade insulation layer;
the outer blade insulation layer is arranged at a proximal end of the tip, the proximal end of the tip being an end of the tip close to the handle;
that the distal end of the sheath is fixedly connected to the outer blade is specifically that the distal end of the sheath is fixedly connected to the outer blade insulation layer;
the inner blade comprises: a support shaft and an inner blade insulation layer;
the inner blade groove is arranged at a distal end of the support shaft; and
the inner blade insulation layer covers a surface of the inner blade except the electrode.

10. The radiofrequency scalpel according to claim 9, further comprising: a sheath insulation layer, the sheath insulation layer covering an edge of the sheath groove and an inner surface of the sheath.

11. The radiofrequency scalpel according to any one of claims 8-10, wherein a sampling gap is reserved between an outer wall of the inner blade and an inner wall of the sheath, and the sampling gap is configured to be capable of serving as a part of an airflow channel of the negative pressure apparatus.

12. A radiofrequency surgical system, comprising a negative electrode plate, and the radiofrequency scalpel according to any one of claims 1-11;
wherein the negative electrode plate and the radiofrequency scalpel are separately a part of a radiofrequency loop.
